# EUROPEAN PATENT SPECIFICATION

(11) **EP 1 439 358 B1**
(45) Date of publication and mention of the grant of the patent: **23.05.2007**
(21) Application number: 03015898.4
(22) Date of filing: 11.07.2003
(51) Int. Cl.: F25D 23/06, A61L 2/23, F25B 47/00

(54) **Refrigerator with a finish material containing nanosilver particles**
Kühlschrank mit einem Silberteilchen im Nanobereich aufweisenden Fertigbearbeitungsmaterial
Réfrigérateur avec un matériau de finissage contenant des nanoparticules d'argent

(30) Priority: 14.01.2003 KR 2003002291; 14.01.2003 KR 2003002292; 14.01.2003 KR 2003002293
(43) Date of publication of application: 21.07.2004
(73) Proprietor: Daewoo Electronics Corporation, Seoul (KR)
(72) Inventor: Jin, Soo Kim, Daewoo Electronics Corporation, Seoul (KR)
(74) Representative: Turi, Michael

(56) References cited:
- EP-A- 0 270 129
- WO-A-00/64259
- DE-A1- 10 120 802
- DE-U1- 20 214 601
- PATENT ABSTRACTS OF JAPAN vol. 1996, no. 12, 26 December 1996 (1996-12-26) & JP 08 210761 A (MATSUSHITA REFRIG CO LTD), 20 August 1996 (1996-08-20)
- PATENT ABSTRACTS OF JAPAN vol. 018, no. 328 (C-1215), 22 June 1994 (1994-06-22) & JP 06 073196 A (HITACHI LTD), 15 March 1994 (1994-03-15)

## Description

The present invention relates to a refrigerator with a finish material containing antibacterial and/or antifungal nanosilver particles; and, more particularly, to a refrigerator including a finish material which contains nanosilver particles exhibiting optimal antibacterial and/or antifungal functions by limiting the size and the concentration of the nanosilver particles to specific ranges which are practical and applicable, respectively.

In general, silver (Ag) is well-known as a common antibacterial agent. Colloidal silver is known as being safe and effective against bacteria, fungi, microbes, virus and the like. Particularly, when silver ions are absorbed into cells of virus, bacteria, fungi and the like, the silver ions prevent the operation of enzyme required in respiration thereof to render them dead. Further, silver ions prevent metabolism of the bacteria and suppress reproductive function thereof.

The fine particles of silver can be produced by a physical process such as electrolysis, liquid phase reduction, and grinding. The electrolysis has mainly been used hitherto in order to obtain stabilized nanosilver particles with a high purity. In the electrolysis process, pure silver (99.99%) is immersed into distilled water; and extremely fine particulates of silver are obtained by applying a low current at a low temperature.

On the other hand, a refrigerator serves to preserve food in a fresh state for a long period in a food storage compartment by cooling air in the compartment using a refrigerating cycle. In the refrigerator, refrigerant gas compressed under a high temperature and a high pressure by a compressor is condensed into liquid phase in a condenser; and the liquid is then pressure-reduced through an expansion valve and evaporated in an evaporator. At this time, the evaporating refrigerant takes heat from ambient air to cool it. The cooled air is then forced to the food storage compartment by, e.g., a fan.

It has been attempted to provide the refrigerator with an antibacterial function. However, in a conventional antibacterial agent such as silver-based zeolite inorganic antibacterial agent and silver powder antibacterial agent with micron-sized particles, the micron-sized silver particles have a specific surface area per a unit weight less than that of nano-sized silver particles; and, therefore, a greater amount of the former is needed than the latter in order to exhibit the same antibacterial ability. Further, the conventional antibacterial agent is not smoothly dispersed while being mixed with a resin since the particle size thereof is large, and is likely to cause scratches to the surface of an injection mold.

DE 202 14 601 U1 discloses an inner part for a refrigerator, which part is coated - at least on its surface - with a antibacterial substance. The antibacterial substance may include silver-zeolite having an average particle size of 1500 nm.

EP 0 270 129 A2 discloses an antibiotic zeolite including silver having an average particle size of 1500 nm.

DE 101 20 802 A1 discloses a method of producing coated nano-sized particles.

WO 00/64259 A1 disclose an ice cooler chest with antimicrobial properties.

It is, therefore, a primary object of the present invention to provide a refrigerator having a finish material containing antibacterial nano-sized silver particles.

It is another object of the present invention to provide a refrigerator with an appropriate antibacterial function by limiting the size and the concentration of the nano-sized silver particles to specific ranges which are suitable for the optimal antibacterial action, respectively, thereby increasing the satisfaction of users.

In accordance with the present invention, there is provided a refrigerator including: a finish material constituting the refrigerator, the finish material containing nano-sized silver particles, wherein the nano-sized silver particles have a size of about 15~300 nm and a concentration of about 50~500 ppm.

The finish material may be an interior finish material which constitutes a food storage compartment of the refrigerator.

The finish material may be formed from a transparent or opaque resin to which pigment is added.

The above and other objects and features of the present invention will become apparent from the following description of preferred embodiments given in conjunction with the accompanying drawings in which:
Fig. 1 is a schematic cross-sectional view of a finish material of the present invention wherein nano-sized silver particles are added thereto;
Figs. 2A and 2B represent antibacterial test results of used test isolates;
Figs. 3A and 3B show test results of MacConkey agar medium cultivation of bacterial in a tangerine in a vessel made of a finish material containing nano-sized silver particles in accordance with the present invention and a conventional vessel made of a finish material without nano-sized silver, respectively; and
Figs. 4A and 4B show test results of MacConkey agar medium cultivation of bacterial in meat in a vessel made of a finish material containing nano-sized silver particles in accordance with the present invention and a conventional vessel made of a finish material without nano-sized silver, respectively.

In the drawings the term "nanosilver" is used to indicate nano-sized silver.

A preferred embodiment of the present invention will now be described in detail with reference to the accompanying drawings.

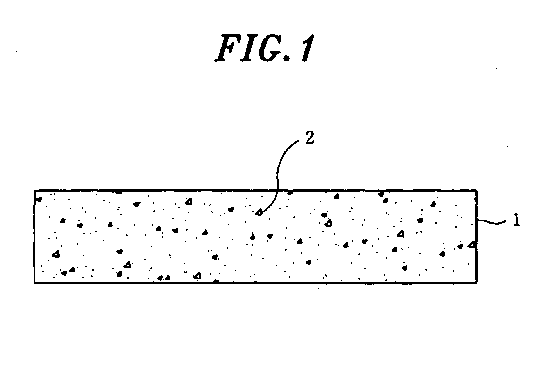

Fig. 1 is a schematic cross-sectional view of a finish material 1 of the present invention wherein nano-sized silver particles 2 are added thereto.

The inventor has studied for employing the nano-sized silver particles so as to provide a refrigerator with an antibacterial function. In other words, by incorporating nano-sized silver particles into a finish material constituting any part of the refrigerator where there may be caused a generation and multiplication of bacteria, such as an interior finish material constituting a food storage compartment of the refrigerator, a storage vessel, a pocket and a part constituting an air circulation duct, the finish material comes to have the antibacterial function.

As shown, the nano-sized silver particles 2 may be contained in the finish material 1, e.g., the interior finish material constituting the food storage compartment of the refrigerator. Alternatively, the nano-sized silver particles 2 may be coated on a surface of the finish material 1 of the refrigerator.

In the preferred embodiment of the present invention, the size of the nano-sized silver particles 2 is limited to about 15~300 nm in consideration of the current manufacturing technique and the efficiency of specific surface area per a unit weight.

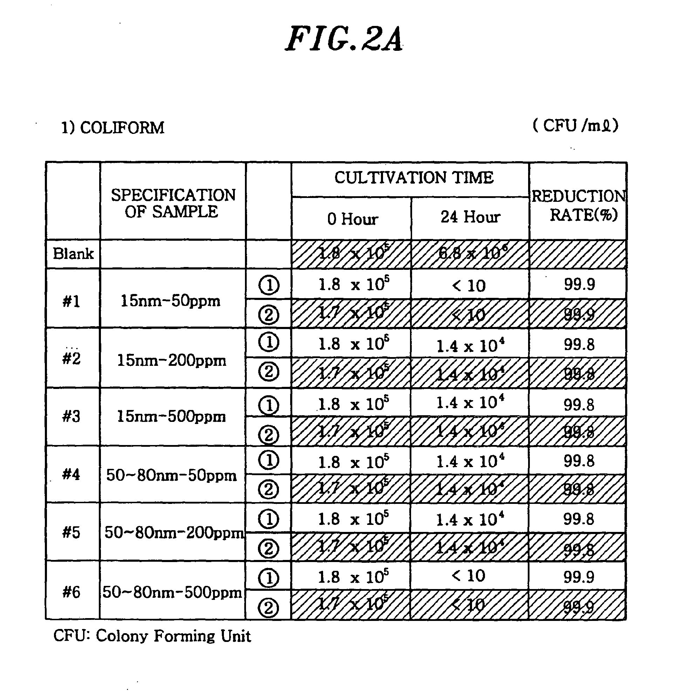

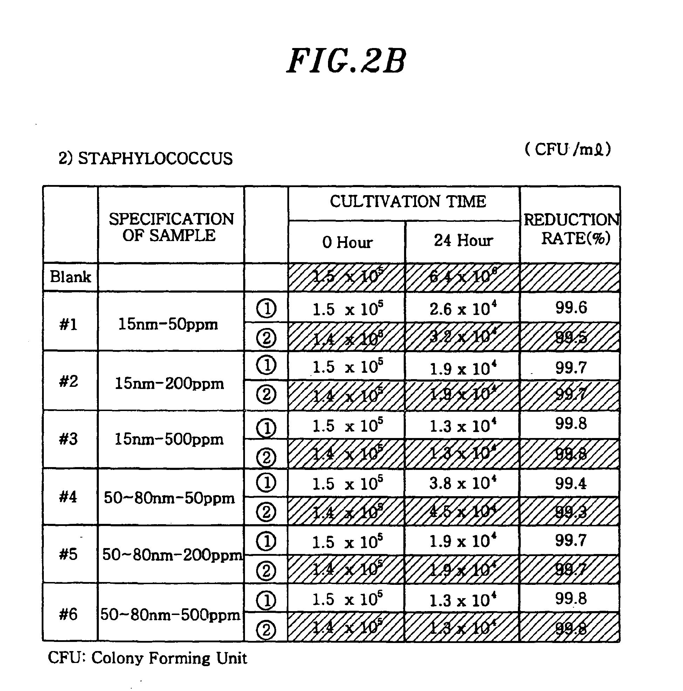

Further, the concentration of the nano-sized silver particles 2 added to the finish material 1 is limited to a range of about 1~500 ppm by weight such that the expensive nano-sized silver can efficiently be used and a target antibacterial ability can be obtained. As the concentration of the nano-sized silver is increased, the antibacterial ability is raised; however, as can be seen from the test results in Figs. 2A and 2B, in the range of 50~500 ppm, there is no significant difference of the antibacterial ability. In addition, in the above concentration range of the nano-sized silver, the sterilization or bacteriostasis can be performed stably and efficiently.

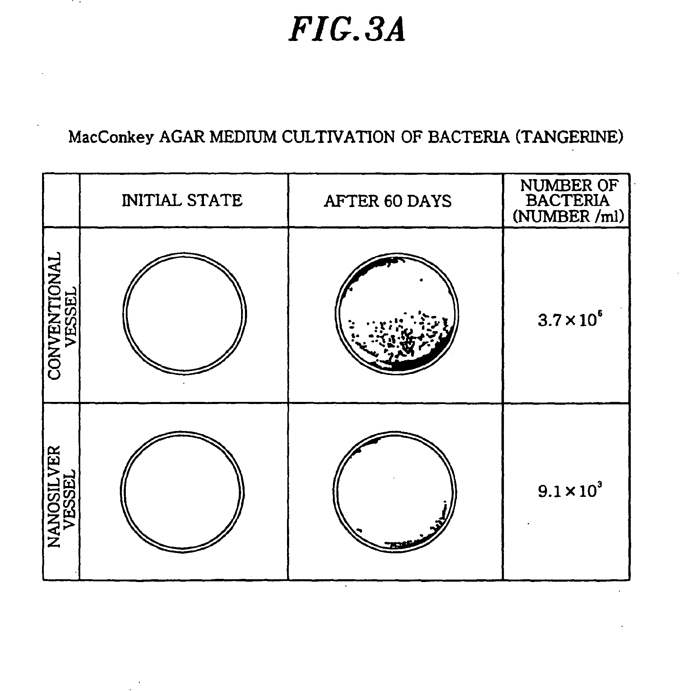

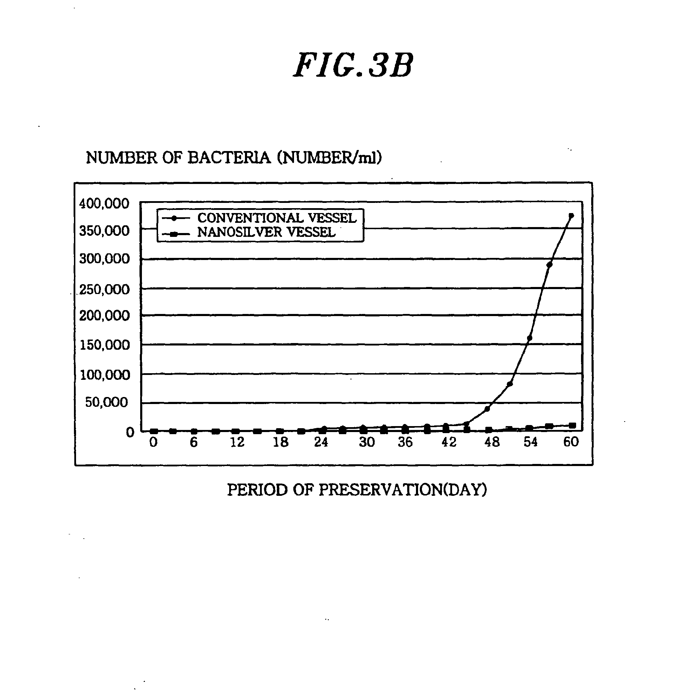

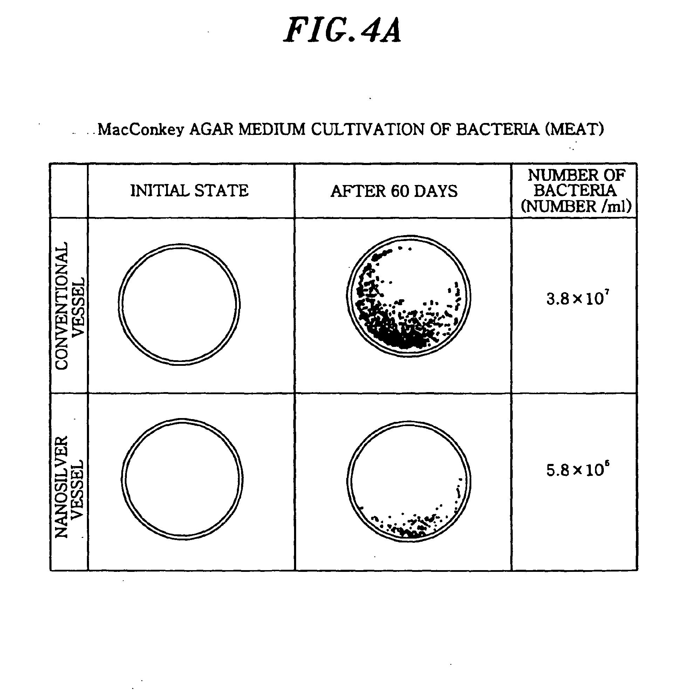

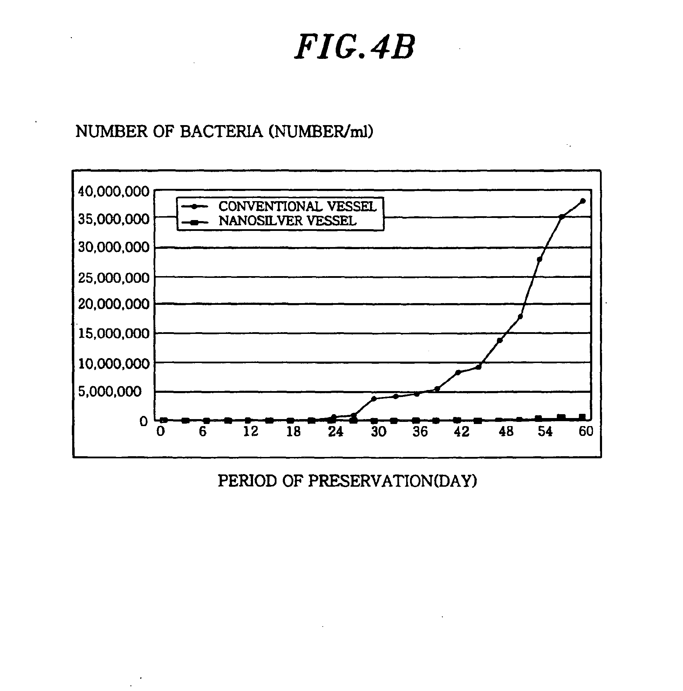

Figs. 3A and 3B show test results of MacConkey agar medium cultivation of bacterial in a tangerine and Figs. 4A and 4B indicate test results in meat. As can be seen from the results, the multiplications of bacteria in the tangerine and the meat are suppressed in a vessel made of the finish material containing nano-sized silver particles in accordance with the present invention more significantly than in a conventional vessel made of a finish material without nano-sized silver.

In addition, the finish material 1 may be made from a transparent or opaque resin to which pigment is added.

## Claims

1. A refrigerator including: a finish material (1) constituting any part of the refrigerator, the finish material (1) containing antibacterial nano-sized silver particles (2),
**characterized in that**:
the nano-sized silver particles (2) have a size of 15~300 nm and a concentration of 50~500 ppm by weight.

2. The refrigerator of claim 1, wherein the finish material (1) is an interior finish material which constitutes a food storage compartment of the refrigerator

3. The refrigerator of claim 1, wherein the finish material (1) is made from a transparent or opaque resin to which pigment is added.

4. The refrigerator of claim 1, wherein the nano-sized silver particles (2) are coated on a surface of the finish material (1).

## Patentansprüche

1. Kühlschrank mit: einem Oberflächenmaterial (1), das einen Teil des Kühlschranks bildet, wobei das Oberflächenmaterial (1) antibakterielle Silberpartikel (2) in Nanogröße enthält, **dadurch gekennzeichnet, dass**:
die Silberpartikel (2) in Nanogröße eine Größe von 15 ~ 300 nm und eine Konzentration von 50 ~ 500 ppm pro Gewicht haben.

2. Kühlschrank nach Anspruch 1, bei dem das Oberflächenmaterial (1) ein inneres Oberflächenmaterial ist, das ein Nahrungsmittelaufbewahrungsfach des Kühlschranks bildet.

3. Kühlschrank nach Anspruch 1, bei dem das Oberflächenmaterial (1) aus einem transparenten oder opaken Harz hergestellt ist, dem Pigment hinzugefügt ist.

4. Kühlschrank nach Anspruch 1, bei dem die Silberpartikel (2) in Nanogröße auf eine Oberfläche des Oberflächenmaterials aufgetragen sind.

## Revendications

1. Réfrigérateur comprenant : un matériau de finition (1) constituant toute partie du réfrigérateur, le matériau de finition (1) contenant des nanoparticules d'argent antibactériennes (2), **caractérisé en ce que** :
les nanoparticules d'argent (2) ont une dimension de 15 à 300 nm et une concentration de 50 à 500 ppm en poids.

2. Réfrigérateur selon la revendication 1, dans lequel le matériau de finition (1) est un matériau de finition intérieure qui constitue un compartiment de stockage alimentaire du réfrigérateur.

3. Réfrigérateur selon la revendication 1, dans lequel le matériau de finition (1) est fabriqué à partir d'une résine transparente ou opaque à laquelle un pigment est ajouté.

4. Réfrigérateur selon la revendication 1, dans lequel les nanoparticules d'argent (2) sont appliquées sur une surface du matériau de finition (1).
